# NEW EUROPEAN PATENT SPECIFICATION

(11) **EP 0 735 850 B2**
(45) Date of publication and mention of the opposition decision: **22.05.2002**
(45) Mention of the grant of the patent: 14.04.1999
(21) Application number: 95905905.6
(22) Date of filing: 15.12.1994
(51) Int. Cl.: A61F 13/15

(54) **SANITARY NAPKIN HAVING CONVEX UPWARD SHAPED CROSS SECTION**
DAMENBINDE MIT EINEM KONVEXEN QUERSCHNITT
SERVIETTE HYGIENIQUE PRESENTANT UNE SECTION CONVEXE VERS LE HAUT

(30) Priority: 22.12.1993 US 171495
(43) Date of publication of application: 09.10.1996
(73) Proprietor: THE PROCTER & GAMBLE COMPANY, Cincinnati, Ohio 45202 (US)
(72) Inventor: BERGMAN, Carl, Louis, Loveland, OH 45140 (US)
(74) Representative: Veronese, Pancrazio
(86) International application number: US9414296
(87) International publication number: WO9517148

(56) References cited:
- WO-A-88/04547
- WO-A-90/14063
- WO-A-91/09579
- WO-A-92/07535
- WO-A-93/01780
- US-A- 3 306 795
- US-A- 4 911 701
- US-A- 5 197 959

## Description

### FIELD OF THE INVENTION

The present invention relates to sanitary napkins, and more particularly sanitary napkins having a means for providing contact with the labia of the wearer.

### BACKGROUND OF THE INVENTION

Sanitary napkins are well known in the art. Sanitary napkins intercept menses upon discharge, and thereby protect the clothing and bedding of the wearer from soiling by the menses. However, oftentimes the sanitary napkin can shift during use and the menses will not hit the target area. Additionally, improved menses interception can occur if the sanitary napkin is in close contact with the body of the wearer.

Several attempts have been made in the art to improve body contact with the wearer, and hence absorb menses upon discharge and thereby minimize soiling by providing a sanitary napkin having a convex upward configuration, particularly in cross section. Examples of such attempts are illustrated in U.S. Patents 2,747,575 issued May 29, 1956 to Mercer; 3,343,543 issued September 26, 1967 to Glassman; 5,171,302 issued December 15, 1992 to Buell; and 5,197,959 issued March 30, 1993 to Buell. However, Glassman suffers from drawbacks which are significant in sanitary napkins successfully commercialized today, and further improvements are possible over Buell:

Mercer requires the sanitary napkin to have stitching through its thickness to achieve a longitudinal medial hump. Stitching a sanitary napkin according to today's construction is simply infeasible due to the materials now utilized. Also this process is prohibitively slow and costly.

Glassman, in particular, requires the sanitary napkin to have longitudinal troughs on the top surface of the sanitary napkin formed by mechanical compression or cutting slits. This sanitary napkin further has a compression formed continuous groove which may or may not include the moisture resistant covering on the back of the sanitary napkin. The groove allows the sanitary napkin to fold into an inverted U-shape. The longitudinal groove, however, does not promote concave cupping of the front of the sanitary napkin around the mons pubis of the wearer and does not fit into the gluteal groove. More importantly, the continuous longitudinal groove prevents the sanitary napkin from having resiliency. Resiliency is the ability of the sanitary napkin to return to an uncompressed configuration when external deformation forces, such as the lateral pressure of the wearer's thighs, are removed. It is important that the sanitary napkin have resiliency for wearer's comfort, and in order that the target area remain as large as possible, and menses does not strike the clothing of the wearer. Furthermore, the compressed groove will have a lower rate of absorbency or a lesser capacity.

The Buell patents teach a sanitary napkin having a deformation element with flexure hinges. The deformation element is a moldable substance such as foam and may be reformable or resilient. The deformation element and hence the sanitary napkin deforms into a W-shaped cross-section in response to lateral pressure from the wearer's thighs. The deformation element, similar to the Mercer stitching, requires an additional component to be added to the sanitary napkin, thus increasing its cost

In yet another attempt, U.S. Patent 5,007,906 issued April 16, 1991 to Osborn, III et al. discloses a sanitary napkin having the topsheet and backsheet attached at one transverse edge and unattached at the other transverse edge. The topsheet and core are separable from the backsheet at the unattached edge. Since the backsheet is attached to the wearer's undergarment, and the core is separable from the backsheet, the core is thereby decoupleable from the backsheet and can better move with the wearer.

Yet other attempts have been made in the art to impart a convex upward cross section to the sanitary napkin to rely upon various elastic means. For example, U.S. Patent 4,911,701 issued March 27, 1990 to Mavinkurve teaches a sanitary napkin having elastic means disposed across the absorbent element and affixed to the impervious backing layer at opposite points on longitudinally extending sides of the sanitary napkin. The elastic means may either be inside or outside the backing layer of the napkin. Likewise, U.S. Patent 5,129,893 issued July 14, 1992 to Thoren discloses a sanitary napkin having elastic means applied to the liquid impermeable surface layer. The elastic means is said to impart resiliency to this sanitary napkin to urge the napkin in its applied condition against the outer genitals of the wearer. However, the attempts by Mavinkurve and Thoren suffer from the drawback that by attaching the elastic to the backsheet, rather than to the core, there is less control over the resulting deformation of the absorbent components of the sanitary napkin and less capability to lift and shape the core and topsheet to the wearer's genitals. Furthermore, attaching the elastic to the backsheet is more likely to cause it to wrinkle, fold, or otherwise detach from the wearer's undergarment. These occurrences either reduce or displace the target area of the sanitary napkin during wear.

International Patent Specification No. WO-A-90/14063 discloses an absorbent article having *inter alia* an outer layer of a highly-absorbent hour glass body and an inner layer of a hose-like body made of a shape-stable liquid permeable material. The inner layer overlies the outer layer being retained thereon by a liquid impervious barrier.

Conversely, directly attaching the elastic to the core, in addition to forming a convex upwards configuration, provides the advantages that the absorbent core can be lifted to the genitals of the wearer further from the backsheet, allowing more intimate contact to occur. Additionally, the absorbent core can be more resiliently shaped to match the shape of the genitals, allowing more intimate contact between the core and the genitals, and sustaining this contact during wear.

Accordingly, it is an object of this invention to provide a sanitary napkin having a core which, through the topsheet, contacts the labia of the wearer. Additionally, it is an object of this invention to provide a sanitary napkin having a convex upwardly shaped core and topsheet Finally, it is an object of this invention to provide a sanitary napkin which has an elastic member attached to the core to provide such convex upwardly shaping.

### SUMMARY OF THE INVENTION

The invention, therefore provides a sanitary napkin in accordance with claim 1 and claims dependent therefrom.

The invention comprises a sanitary napkin having mutually orthogonal longitudinal and lateral centerlines. The sanitary napkin comprises a liquid pervious topsheet, a liquid impervious backsheet, and an absorbent core intermediate the topsheet and the backsheet. The absorbent core has alternately disposed longitudinal side edges and transverse end edges, as does the periphery of the sanitary napkin. Joined to the absorbent core is at least one elastic member for foreshortening the sanitary napkin in the lateral direction. The elastic member is joined to the absorbent core and spans the longitudinal centerline. The elastic member is preferably disposed intermediate the absorbent core and the backsheet. The elastic member laterally foreshortens the sanitary napkin to a convex upward configuration.

### BRIEF DESCRIPTION OF THE DRAWINGS

Figure 1 is a top plan view of a sanitary napkin according to the present invention, shown partially in cutaway.
Figure 2 is a vertical sectional view of a convex upwardly shaped cross section of the sanitary napkin.
Figure 3 is an exploded perspective view of a sanitary napkin wherein the elastic member wraps the edges of the core of a sanitary napkin.

### DETAILED DESCRIPTION OF THE INVENTION

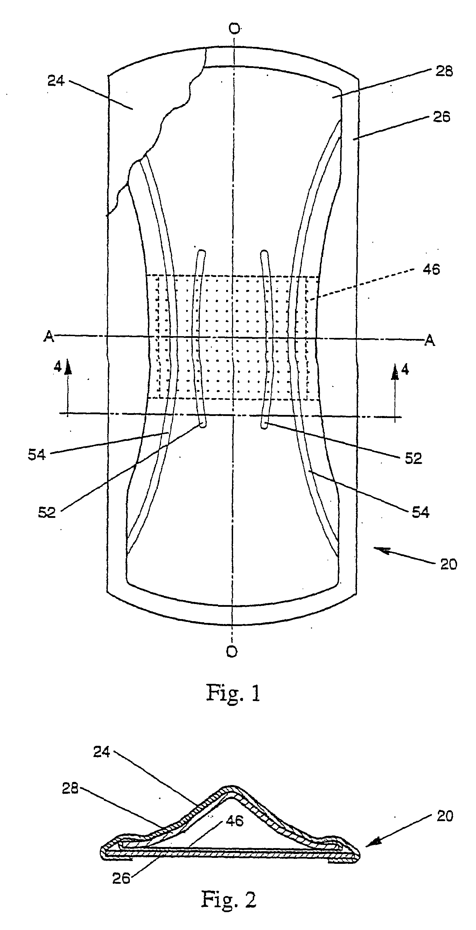

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use, and, preferably, to be recycled, composted or otherwise disposed of in an environmentally compatible manner). A "unitary" absorbent article refers to absorbent articles which are formed of separate parts united together to form a coordinated entity so that they do not require separate manipulative parts like a separate holder and pad. A preferred embodiment of a unitary disposable absorbent article of the present invention is the catamenial pad, sanitary napkin 20, shown in Figure 1. As used herein, the term "sanitary napkin" refers to an absorbent article which is worn by females adjacent to the pudendal region, generally external to the urogenital region, and which is intended to absorb and contain menstrual fluids and other vaginal discharges from the wearer's body (e.g., blood, menses, and urine). Interlabial devices which reside partially within and partially external of the wearer's vestibule are also within the scope of this invention. It should be understood, however, that the present invention is also applicable to other feminine hygiene or catamenial pads such as panty liners, or other absorbent articles such as incontinence pads, and the like.

Figure 1 is a plan view of the sanitary napkin 20 of the present invention in its flat-out state with portions of the structure being cut-away to more clearly show the construction of the sanitary napkin 20 and with the portion of the sanitary napkin 20 which faces or contacts the wearer, oriented towards the viewer. As shown in Figure 1, the sanitary napkin 20 preferably comprises a liquid pervious topsheet 24, a liquid impervious backsheet 26 joined to the topsheet 24, and absorbent core 28 intermediate the topsheet 24 and the backsheet 26. The absorbent core 28 has two major faces, a first major face oriented towards the topsheet 24 and a second major face oriented towards the backsheet 26. The first major face is on the tension side of the absorbent core 28 when it is deformed to a convex upwards configuration as illustrated in Figures 2 and 4.

Convex upwards configurations are inclusive of, but not limited to, inverted U-shapes or inverted V-shapes. Joined to the core 28 and spanning the longitudinal centerline O-O is an elastic member 46 which imparts the convex upwards configuration.

The sanitary napkin 20 has two centerlines, a longitudinal centerline O-O and a lateral centerline A-A orthogonal thereto. The term "longitudinal" as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The term "longitudinally oriented" refers to a direction within ± 45 degrees of the longitudinal direction. The terms "transverse" or "lateral" as used herein, are interchangeable, and refer to a line, axis or direction which lies within the plane of the sanitary napkin 20 and is generally perpendicular to the longitudinal direction. The Z-direction is orthogonal both the longitudinal and lateral centerlines O-O and A-A of the sanitary napkin 20 and extends outwardly from the plane of the sanitary napkin 20, which is defined by the longitudinal centerline O-O and lateral centerline A-A.

The long edges of the sanitary napkin 20, which are aligned with the longitudinal centerline O-O, are the longitudinal side margins of the sanitary napkin 20. The ends of the sanitary napkin 20 joining the longitudinal side margins are the transverse ends of the sanitary napkin 20. Collectively the longitudinal side margins and transverse ends of the sanitary napkin 20 define its periphery. Similarly, the core 28 of the sanitary napkin has a periphery defined by alternately disposed longitudinal side margins and transverse ends.

While the topsheet 24, the backsheet 26, and the absorbent core 28 may be assembled in a variety of well known configurations (including so called "tube" products or side flap products), preferred sanitary napkin configurations are described generally in U.S. Patent 4,950,264, "Thin, Flexible Sanitary Napkin" issued to Osborn on August 21, 1990; U.S. Patent 4,425,130, "Compound Sanitary Napkin" issued to DesMarais on January 10, 1984; U.S. Patent 4,321,924, "Bordered Disposable Absorbent Article" issued to Ahr on March 30, 1982; U.S. Patent 4,589,876, "Shaped Sanitary Napkin With Flaps" issued to Van Tilburg on August 18, 1987. Figure 1 shows a preferred embodiment of the sanitary napkin 20 in which the topsheet 24 and the backsheet 26 have length and width dimensions generally larger than those of the absorbent core 28. The topsheet 24 and the backsheet 26 extend beyond the edges of the absorbent core 28 to thereby form not only portions of the periphery but also side flaps.

The absorbent core 28 may be any absorbent means capable of absorbing or retaining liquids (e.g., menses and/or urine). The absorbent core 28 may be manufactured in a wide variety of sizes and shapes (e.g., rectangular, oval, hourglass, dog bone, asymmetric, etc.) and from a wide variety of liquid-absorbent materials commonly used in sanitary napkins and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; synthetic fibers such as crimped polyester or polyolefin fibers; peat moss; tissue including tissue wraps and tissue laminates; absorbent foams; absorbent sponges; superabsorbent polymers; absorbent gelling materials; or any equivalent material or combinations of materials, of mixtures of these. Prophetically, particularly preferred absorbent core 28 is made of thermally bonded air laid material having approximately 15 percent synthetic fibers. Synthetic fibers are preferred due to the ease with which they fuse together to join the core 28 and topsheet 24 as described below. A particularly suitable synthetic fiber is a bi-component material having a polyethylene sheath and a polypropylene center.

The configuration and construction of the absorbent core 28 may also be varied (e.g., the absorbent core 28 may have varying caliper zones (e.g., profiled so as to be thicker in the center), hydrophilic gradients, superabsorbent gradients, or lower density and lower average basis weight acquisition zones; or may comprise one or more layers or structures). The total absorbent capacity of the absorbent core 28 should, however, be compatible with the design loading and the intended use of the sanitary napkin 20. Further, the size and absorbent capacity of the absorbent core 28 may be varied to accommodate different uses such as incontinence pads, panty liners, regular sanitary napkins, or overnight sanitary napkins.

Preferably the absorbent core 28 is made of air laid tissue laminate having a wet tensile strength in the transverse direction of at least 100 grams per centimeter as determined by ASTM Standard D829-49. Such a wet tensile strength is necessary in order for the core 28 to withstand the forces imposed by the elastic member 46 while the sanitary napkin 20 is in use. In this regard, a core 28 made of an air laid tissue has generally been found to work better than a fluff or airfelt core 28. However, the tensile strength of the core 28 should not be too great, otherwise the sanitary napkin 20 according to the present invention may not have the requisite flexibility. Preferably the sanitary napkin 20 according to the present invention has a flexibility of less than 300 grams, and more preferably, less than 100 grams as measured in accordance with the aforementioned U.S. Patent 4,950,264 issued to Osborn.

The sanitary napkin 20 according to the present invention is preferably decoupleable. In this arrangement and as illustrated by Figure 2, the core 28 is not joined to the backsheet 26, particularly at and near the longitudinal centerline O-O. This arrangement allows the core 28 to separate from the backsheet 26 in the Z-direction in a process known as "decoupling." Of course, if the core 28 separates from the backsheet 26, it is necessary that the topsheet 24 separate from the core 28 as well. By decoupling the core 28 from the Backsheet 26 in the Z-direction, the core 28 can remain in contact with the wearer so that it is more likely to intercept menses upon discharge, while the backsheet 26 moves with the undergarment of the wearer. More preferably, the sanitary napkin 20 may have longitudinally extending pleats, limiting the amount of Z-direction decoupling of the core 28 and backsheet 26.

A decoupleable sanitary napkin 20 and having such pleats may be made in accordance with commonly assigned U.S. Patent 5,007,906 issued April 16, 1991 to Osborn, III et al. A particularly preferred decoupleable sanitary napkin 20 according to the present invention has a lateral pleat on the detached rear end of the sanitary napkin 20.

A decoupleable sanitary napkin 20 having an elastic member 46 is particularly preferred because in this execution the core 28 is longitudinally decoupled from and can longitudinally move relative to the backsheet 26. and hence relative to the wearer's undergarment to which the backsheet 26 is attached. Such decoupling is highly desirable because the backsheet 26 must have a different radius of curvature (as measured in the longitudinal direction) than the core 28 if the backsheet 26 is to remain connected with the undergarment while the core 28 and topsheet 24 are in intimate contact with the body of the wearer. Longitudinal decoupling of the sanitary napkin 20, in conjunction with Z-direction decoupling suitably accommodates the shear forces caused by this difference in radii of curvature.

Preferably the core 28 is joined to the topsheet 24. Adhesive joining is suitable and commonly used in the art Joining the core 28 and topsheet 24 ensures these components will move as an integral unit, so that any menses deposited on the topsheet 24 is more readily absorbed by the core 28.

The backsheet 26 is impervious to liquids and is preferably manufactured from a thin plastic film, although other flexible liquid impervious materials may also be used. As used herein, the term "flexible" refers to materials which are compliant and will readily conform to the general shape and contours of the human body. The backsheet 26 prevents exudates absorbed and contained in the absorbent core 28 from wetting the clothing and bedding of the wearer. The backsheet 26 may alternatively comprise a film-coated nonwoven material. Preferably, the backsheet 26 is a polyethylene film having a thickness of from about 0.012 mm (0.5 mil) to about 0.051 mm (2.0 mils). Exemplary polyethylene films are manufactured by Clopay Corporation of Cincinnati, Ohio, under the designation 161-0001, and by Tredegar Corporation, of Terre Haute, Indiana, under the designation XP-39385.

The backsheet 26 has an inwardly oriented surface oriented towards the absorbent core 28 and an outwardly oriented surface opposed thereto. The outwardly oriented surface of the backsheet 26 faces, and in use contacts, the undergarment of the wearer. If desired, strips of adhesive may be joined to the outwardly facing surface of the backsheet 26 to facilitate attachment of the sanitary napkin 20 to the undergarment of the wearer.

Referring back to Figure 1, the topsheet 24 is compliant, soft feeling, and non-irritating to the wearer's skin. Further, the topsheet 24 is liquid pervious permitting liquids (e.g., menses and/or urine) to readily penetrate through its thickness. A suitable topsheet 24 may be manufactured from apertured formed films, foams, or woven and nonwoven materials comprised of natural or synthetic fibers. A preferred topsheet 24 comprises an apertured formed film as described in U.S. Patent 3,929,135, entitled "Absorptive Structures Having Tapered Capillaries," which issued to Thompson on December 30, 1975; U.S. Patent 4,324,246 entitled "Disposable Absorbent Article Having A Stain Resistant Topsheet," which issued to Mullane, et al. on April 13, 1982; U.S. Patent 4,342,314 entitled "Resilient Plastic Web Exhibiting Fiber-Like Properties," which issued to Radel et al. on August 3, 1982: U.S. Patent 4,463,045 entitled "Macroscopically Expanded Three-Dimensional Plastic Web Exhibiting Non-Glossy Visible Surface and Cloth-Like Tactile Impression, which issued to Ahr et al. on July 31, 1984; and U.S. Patent 5,006,394 entitled "Multilayer Polymeric Film," which issued to Baird on April 9, 1991.

The preferred topsheet 24 for the present invention is the formed film described in one or more of the above patents and marketed by The Procter & Gamble Company of Cincinnati, Ohio as "DRI-WEAVE" for sanitary napkins 20. For the invention described and claimed herein, it is preferred that the topsheet 24 be synthetic, so that the topsheet 24 can be joined to synthetic fibers of the absorbent core 28 by heat. For the embodiments described herein, the topsheet 24 and the core 28 may be joined at continuous or discontinuous lines of weakness 30, 30' using embossing plates having a surface temperature of about 149 to 177 degrees C. The embossing plates may be used to apply pressure to the topsheet 24 and core 28, each having synthetic materials, for about 10 seconds.

The backsheet 26 and the topsheet 24 are positioned adjacent the first and second major faces of the core 28 respectively, so that the core 28 is intermediate the backsheet 26 and topsheet 24. The backsheet 26 and topsheet 24 are preferably joined thereto and to each other by attachment means (not shown) such as are well known in the art. Adhesives which have been found to be satisfactory attachment means are manufactured by H. B. Fuller Company of St Paul, Minnesota under the designation HL-1258 or H-2031. The attachment means will preferably comprise an open pattern network of filaments of adhesive as is disclosed in U.S. Patent 4,573,986 entitled "Disposable Waste-Containment Garment," which issued to Minetola et al. on March 4, 1986, and which is incorporated herein by reference. Alternative attachment means comprise heat, pressure, ultrasonic, and dynamic mechanical bonds.

In an afternative embodiment of the present invention, the sanitary napkin 20 has two flaps (not shown), each of which extend laterally beyond the longitudinal side margins of the sanitary napkin 20. The flaps are configured to drape over the edges of the wearer's undergarment. The flaps help prevent soiling by menstrual fluid, preferably by forming a double walled barrier along the edges of the undergarment The flaps are preferably provided with attachment means on the garment surface, so that they can be folded back underneath the crotch of the undergarment in this way the flaps serve to keep the sanitary napkin 20 properly positioned.

The flaps may be constructed of various materials including materials used for the topsheet 24, backsheet 26. combinations thereof, and may be a laminate having tissue in the center. Further, the flaps may be a separate element attached to the main body of the sanitary napkin 20 or can comprise extensions of the topsheet 24 and/or backsheet 26. Preferred flaps may be made in accordance with U.S. Patents 4,589,876 issued May 20, 1986 to Van Tilburg or 4,687,478 issued August 18, 1987 to Van Tilburg.

The elastic member 46 is preferably generally disposed intermediate the core 28 and the backsheet 26, so that the backsheet facing side of the core 28 is contracted and forms a shape concave towards the backsheet 26, thus causing the topsheet facing side of the core 28 to form a convex shape relative to the topsheet 24 and the genitals of the wearer. The elastic member 46 may be generally parallel the lateral axis A-A, or should at least have a vector component parallel thereto.

The elastic member 46 must have a transverse contractive force sufficient to impart a convex upward cross section to the core 28. Preferably, the elastic member 46 imparts a contractive force sufficient to decouple the core 28 from the backsheet 26. Preferably, the elastic member 46 is generally longitudinally centered, i.e., disposed near the lateral centerline A-A so that the contractive force is relatively evenly distributed towards both transverse ends of the core 28.

A suitable elastic member 46 preferably has an elastic extensibility of at least about 50 percent, and is provided in the form of a flat film. Preferably, a flat film elastic member 46 has a width in the longitudinal direction of at least about 10-30 millimeters, in order to avoid imposing an undue stress concentration at a particular location of the absorbent core 28. The elastic film should further have a minimal thickness in the Z-direction, in order to avoid discomfort to the wearer. A suitable elastic member 46 may be made of a heat-shrink type film, such as Extra-flex PGO-423 available from the Exxon Company of Houston, Texas. Such a film, having a width in the longitudinal direction of about 20 millimeters, and applied at a transverse dimension of about 30 millimeters prior to heat shrinking about 10 to about 25 percent has been found to work well for the embodiments described herein utilizing an air laid tissue core 28.

The elastic member 46 should be sized to provide enough lift of the core 28 from the backsheet 26 to ensure contact of the topsheet 24 with the labia of the wearer. However, the elastic member 46 should not provide too much lift, otherwise the sanitary napkin 20 will be uncomfortable to the wearer. In a preferred embodiment the convex shape of the sanitary napkin 20 will flatten to a generally planar configuration under a pressure of about 0.2 to about 0.3 pounds per square inch.

The elastic member 46 may be joined to the core 28 by autogenous bonding as described in commonly assigned U.S. Patent 4,854,984 issued August 8, 1989 to Ball et al. Alternatively, the elastic member 46 may be joined to the core 28 by ultrasonic bonding at discretely spaced bond sites or by using adhesive, as is well known in the art A suitable adhesive is double-sticky tape available from Anchor Continental, 3 Sigma Division of Covington, Ohio. Ultrasonic bonding and adhesive bonding are generally preferred to attach the elastic member 46 to the absorbent core 28. This preference results from the localized strengthening which occurs due to the sites of attachments of the ends of the elastic member 46.

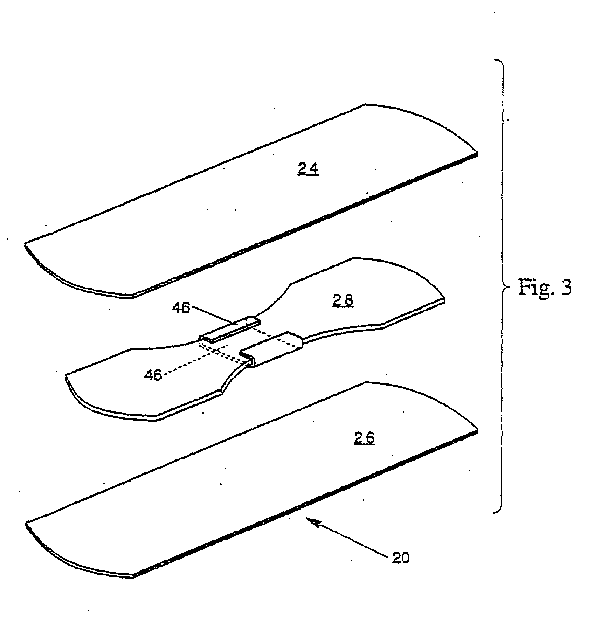

Referring to Figure 3, the ends of the elastic member 46 wraps the edges of the core 28 to provide better attachment.

Referring back to Figure 1, if desired, although not part of the present invention, the sanitary napkin may be provided with lines of weakness 52, 54. The lines of weakness 52, 54 may be formed by embossing the core 28 and may optimally further be embossed through the topsheet 24. This arrangement provides lines of weakness 52, 54 in the topsheet 24 registered with the lines of weakness 52, 54 in the core 28.

The lines of weakness 52, 54 are preferably symmetrically disposed about the longitudinal axis. The two inboard lines of weakness 52 are preferably concave towards the longitudinal centerline O-O. The two outboard lines of weakness 54 are preferably convex outward from the longitudinal centerline O-O. This arrangement allows the sanitary napkin 20 to preferentially deform, in response to lateral pressure from the thighs of the wearer and the contractive force imparted by the elastic member 46.

In this arrangement, the ends of, or at least the outboard attachment points of the elastic member 46, are joined to the core 28 intermediate the lines of weakness 52, 54.

Of course, many variations of the claimed invention are feasible. Additionally, a segmented core 28 may be utilized having different attachment points for the plural elastic members. One elastic member 46 spans the entire lateral dimension of the core 28, while other elastic member(s) span only a portion of the core 28.

It is to be recognized all such variations are within the scope of the claimed invention.

## Claims

1. A sanitary napkin (20) having a longitudinal centerline (O) and a lateral centerline (A) orthogonal thereto and defining longitudinal and lateral directions respectively, said napkin (20) comprising:
a liquid pervious topsheet (24);
a liquid impervious backsheet (26) at least partially peripherally joined to said topsheet (24);
an absorbent core (28) intermediate said topsheet (24) and said backsheet (26);
at least one elastic member (46) for foreshortening said napkin (20); said elastic member (46) being disposed on opposite sides of said longitudinal centerline (O) and extending laterally thereacross so that said elastic member (46) spans said longitudinal centerline (O), whereby said elastic member (46) laterally foreshortens said napkin (20) to provide a convex upwardly shaped configuration, wherein said elastic member (46) is directly joined to said absorbent core (28) at attachment positions disposed on said opposite sides and applies an elastic contractive force to the core
**characterised in that**
said elastic member (46) is disposed between said core (28) and said backsheet (36);
said core (28) has longitudinal side margins;
said elastic member (46) is joined to said core (28) at said longitudinal side margins; and
said elastic member (46) wraps said longitudinal side margins of said core (28).

2. A napkin (20) as claimed in Claim 1 wherein said core (28) is divided into a plurality of longitudinally spaced independent segments (28', 28'', 28''', 28'''') ;
preferably further comprising a plurality of elastic members (46), wherein each elastic member (46) is joined to a different segment (28', 28'', 28''', 28'''') of said core (28), and more preferably said plural elastic members (46) apply different contractive forces to the respective segments (28', 28'', 28''', 28'''') of said core (28) to which said elastic member (46) is joined.

3. A napkin (20) as claimed in Claim 1 or Claim 2 wherein said core (28) is decoupleable from said backsheet (36) in the Z-direction at said longitudinal centerline (10).

4. A napkin as claimed in any of Claims 1-3 wherein the elastic member (46) has an elastic extensibility of at least 50%.

5. A napkin as claimed in any of Claims 1-4 wherein the elastic contractive force is sufficient to decouple the core (28) from the backsheet (26).

## Patentansprüche

1. Eine Hygienevorlage (20) mit einer Längsmittellinie (0) und einer dazu orthogonalen Quermittellinie (A), Längs- bzw. Querrichtungen definierend, wobei die genannte Vorlage (20) umfaßt:
ein flüssigkeitsdurchlässiges Deckblatt (24);
ein flüssigkeitsundurchlässiges Rückenblatt (26), welches mindestens teilweise peripher mit dem genannten Deckblatt (24) verbunden ist;
einen absorbierenden Kern (28) zwischen dem genannten Deckblatt (24) und dem genannten Rückenblatt (26);
mindestens einen elastischen Bauteil (46) zum Verkürzen der genannten Vorlage (20); wobei der genannte elastische Bauteil (46) an gegenüberliegenden Seiten der genannten Längsmittellinie (0) angeordnet ist und sich quer darüber erstreckt, sodaß der genannte elastische Bauteil (46) die genannte Längsmittellinie (0) überspannt, wodurch der genannte elastische Bauteil (46) die genannte Vorlage (20) quer verkürzt, um eine konvex aufwärts geformte Konfiguration beizustellen, wobei der genannte elastische Bauteil (46) mit dem genannten absorbierenden Kern (28) an Fixierungspositionen, welche an den genannten gegenüberliegenden Seiten angeordnet sind, direkt verbunden ist und eine elastische Kontraktionskraft auf den Kern aufbringt,
**dadurch gekennzeichnet, daß** der genannte elastische Bauteil (46) zwischen dem genannten Kern (28) und dem genannten Rückenblatt (26) angeordnet ist;
daß der genannte Kern (28) Längsseitenrandstreifen aufweist;
daß der genannte elastische Bauteil (46) mit dem genannten Kern (28) an den genannten Längsseitenrandstreifen verbunden ist; und
daß der genannte elastische Bauteil (46) die genannten Längsseitenrandstreifen des genannten Kerns (28) umhüllt.

2. Eine Vorlage (20), wie sie in Anspruch 1 beansprucht ist, bei welcher der genannte Kern (28) in eine Mehrzahl von der Länge nach beabstandeten unabhängigen Segmenten (28', 28", 28''', 28'''') unterteilt ist;
vorzugsweise weiters eine Mehrzahl von elastischen Bauteilen (46) umfassend, wobei jeder elastische Bauteil (46) mit einem unterschiedlichen Segment (28', 28", 28"', 28'''') des genannten Kerns (28) verbunden ist und bevorzugter die genannten mehreren elastischen Bauteile (46) unterschiedliche Kontraktionskräfte auf die jeweiligen Segmente (28', 28", 28''', 28'''') des genannten Kerns (28), mit welchen der genannte elastische Bauteil (46) verbunden ist, aufbringen.

3. Eine Vorlage (20), wie sie in einem der Ansprüche 1 bis 2 beansprucht ist, bei welcher der genannte Kern (28) vom genannten Rückenblatt (26) in der Z-Richtung an der genannten Längsmittellinie (0) entkuppelbar ist.

4. Eine Vorlage (20), wie sie in einem der Ansprüche 1 bis 3 beansprucht ist, bei welcher der genannte elastische Bauteil (46) eine elastische Verlängerbarkeit von mindestens 50 % aufweist.

5. Eine Vorlage, wie sie in einem der Ansprüche 1 bis 4 beansprucht ist, bei welcher die elastische Kontraktionskraft ausreichend ist, um den Kern (28) vom Rückenblatt (26) zu entkuppeln.

## Revendications

1. Serviette hygiénique (20) présentant une ligne médiane longitudinale (O) et une ligne médiane latérale (A) orthogonale à celle-ci, et définissant respectivement des directions longitudinale et latérale, ladite serviette (20) comprenant:
une feuille de dessus (24) perméable aux liquides;
une feuille de fond (26) imperméable aux liquides réunie au moins partiellement de manière périphérique à ladite feuille de dessus (24);
une âme absorbante (28) située entre ladite feuille de dessus (24) et ladite feuille de fond (26);
au moins un élément élastique (46) destiné à raccourcir ladite serviette hygiénique (20), ledit élément élastique (46) étant placé sur des côtés opposés de ladite ligne médiane longitudinale (O) et s'étendant latéralement en travers de celle-ci afin que ledit élément élastique (46) s'étende sur ladite ligne médiane longitudinale (O), de sorte que ledit élément élastique (46) raccourcit latéralement ladite serviette (20) afin de fournir une configuration convexe profilée vers le haut, dans laquelle ledit élément élastique (46) est directement réuni à ladite âme absorbante (28) au niveau de positions de fixation placées sur lesdits côtés opposés, et applique une force élastique de contraction à l'âme
**caractérisée en ce que**
ledit élément élastique (46) est placé entre ladite âme (28) et ladite feuille de fond (36);
ladite âme (28) a des bords latéraux longitudinaux;
ledit élément élastique (46) est réuni à ladite âme (28) au niveau des bords latéraux longitudinaux; et
ledit élément élastique (46) enveloppe lesdits bords latéraux longitudinaux de ladite âme (28).

2. Serviette (20) selon la revendication 1, dans laquelle ladite âme (28) est divisée en une pluralité de segments indépendants espacés longitudinalement (28', 28", 28''', 28"");
comprenant en outre, de préférence, une pluralité d'éléments élastiques (46), dans laquelle chaque élément élastique (46) est réunie à un segment différent (28', 28", 28"', 28"") de ladite âme (28) et, plus préférablement, ladite pluralité d'éléments élastiques (46) applique différentes forces de contraction aux segments respectifs (28', 28", 28"', 28"") de ladite âme (28) à laquelle ledit élément élastique (46) est réuni.

3. Serviette (20) selon la revendication 1 ou la revendication 2, dans laquelle ladite âme (28) peut être découplée de ladite feuille de fond (36) dans la direction Z au niveau de ladite ligne médiane longitudinale (10).

4. Serviette selon l'une quelconque des revendications 1-3, dans laquelle l'élément élastique (46) a une extensibilité élastique d'au moins 50 %.

5. Serviette selon l'une quelconque des revendications 1-4, dans laquelle la force élastique de contraction est suffisante pour découpler l'âme (28) de la feuille de fond (26).
